# EUROPEAN PATENT APPLICATION

(11) **EP 1 048 203 A2**
(43) Date of publication of application: **02.11.2000**
(21) Application number: 00303445.1
(22) Date of filing: 25.04.2000
(51) Int. Cl.: A01H 4/00

(54) **Method for producing plantlets of sweet potato**

(30) Priority: 27.04.1999 JP 11956999
(71) Applicant: Nisshinbo Industries, Inc., Chuo-ku, Tokyo 103-0013 (JP)
(72) Inventor: Hasegawa, Osamu c/o Nisshinbo Ind.,Inc., R&D Cter., Chiba, 267-0056 (JP); Ohno, Yohjiroh, c/o Nisshinbo Ind.,Inc., R&D Cter., Chiba, 267-0056 (JP); Kubota, Chieri, Chiba, 271-0092 (JP); Zobayed, Fawzia Afreen, c/o Dpt. of Horticulture o, Chiba, 271-0092 (JP)
(74) Representative: Bannerman, David Gardner

(57) **Abstract**

Explants of sweet potato are cultured by using a support material comprising vermiculite and cellulose fibers, preferably in a weight ratio of 5:95 to 95:5, preferably in a medium not containing a saccharide, while maintaining carbon dioxide gas concentration in culture environment to be 400 to 3,000 µmol/mol, to produce plantlets of sweet potato, that show excellent growth and have storage roots formed during the culture. Thus, there is provided a method for producing plantlets of sweet potato that show excellent growth and have storage roots.

## Description

### BACKGROUND OF THE INVENTION

### Field of the Invention

The present invention relates to a method for producing plantlets of sweet potato. More precisely, it relates to a method for producing plantlets of sweet potato with formed storage roots.

### Description of the Related Art

Sweet potato is a plant utilized for various purposes, for example, as food, fuel alcohol resources and so forth. Therefore, it is expected to play a very important role to solve future problems relating to food and energy/resource shortages, environmental protection and so forth.

The current methods for producing sweet potato in a greater amount include methods utilizing seedlings obtained from cuttings or seedlings budding from seed potatoes. However, because propagation rates attained by these methods are low, these methods will not be able to accommodate enormous increase in demand of seedlings, which is expected for sweet potato seedlings. Therefore, sweet potato seedlings produced by tissue culture (micropropagation), which is referred to as "plantlets " hereafter, have become an object of research attention.

In general, the production of plantlets by cell culture technology can be divided roughly into the following stages; stage of selecting and preparing plants for propagation (Stage 0), stage of establishing sterile culture (Stage 1), stage of allowing propagation (Stage 2), stage of allowing rooting in vitro and conditioning (Stage 3), and stage of acclimatization in an ex vitro environment (Stage 4). As methods for culturing such plantlets, there have been most commonly known culture methods utilizing a gelling agent such as agar for support materials.

When a gelling agent such as agar is used, a saccharide (sucrose, glucose, fructose etc.) is added to a medium as a carbon source. This makes the medium more susceptible to external bacterial contamination during the culture period. In order to prevent such contamination, extreme care is required in handling, in order to maintain a sterile condition for culture vessels and culture rooms in which the vessels are stored.

Furthermore, root growth is retarded, as air cannot enter into the agar medium. As well, even if rooting is improved by use of an agar medium specially suited for better rooting, growing roots do not function properly as they exhibit characteristics of aquatic roots, and ground parts of such seedlings cannot survive when these seedlings are directly planted in nursery soil. Therefore, after being removed from the agar, the seedlings are acclimatized in a support medium comprising perlite, vermiculite or the like, and then transplanted into nursery soil, either in a open field or in a greenhouse. However, this acclimatization process takes a long period of time, i.e., 1 to 3 months. In addition, there may be observed poor survival during acclimatization. That is, there may be observed death of the plantlets, and seedlings may remain in a submerged shape, which means the seedling may not have a normal shape. Therefore, the method suffers from a number of problems concerning labor and production yield.

On the other hand, there has been reported another method for propagating sterile sweet potato seedlings with very little bacterial contamination during culture period by utilizing rock-wool or vermiculite as a support material under a sterilized condition with no sugar (Japanese Patent Laid-open (Kokai) No. 9-294495).

However, when plantlets of sweet potato are produced by the above method, produced plantlets do not form storage roots that is to eventually grow into potatoes, but storage root formation begins anew after the seedlings are transplanted in nursery soil. Therefore, this method still requires improvement in order to accelerate potato maturation.

By the way, it has also been reported that the usage of a plant tissue culture medium containing vermiculite and cellulose fibers results in very good rooting, thus it enables to obtain healthy seedlings that do not require acclimatization after the culture and such seedlings can then be transplanted in open field nursery soil as they are (WO97/48271). However, it has not been known whether plantlets with storage roots can be obtained when this technique is applied to sweet potato. In addition, there has been known no concept of storage root formation of sweet potato plantlets during culture process.

### SUMMARY OF THE INVENTION

The present invention has been accomplished in view of the above points, and an object of the present invention is to provide a method for producing plantlets of sweet potato with formed storage roots that eventually grow into sweet potatoes by performing tissue culture of explants of sweet potato.

The inventors of the present invention assiduously studied in order to achieve the aforementioned object. As a result, they found that plantlets of sweet potato obtained via tissue culture of explants of sweet potato by using a support material comprising vermiculite and cellulose fibers show good growth and have storage roots on their roots, and thus accomplished the present invention.

That is, the present invention provides a method for producing plantlets of sweet potato with formed storage roots, wherein tissue culture of explants of sweet potato is performed by using a support material comprising vermiculite and cellulose fibers.

The present invention also provides the aforementioned method for producing plantlets of sweet potato, wherein vermiculite and cellulose fibers are used in a weight ratio of 5:95 to 95:5.

The present invention further provides the aforementioned method for producing plantlets of sweet potato, wherein the culture is performed in a medium not containing a saccharide.

The present invention still further provides the aforementioned method for producing plantlets of sweet potato, wherein the culture is performed while maintaining carbon dioxide gas concentration in culture environment to be 400 to 3,000 µmol/mol.

### BRIEF DESCRIPTION OF THE DRAWINGS

Other objects and advantages of the present invention will become apparent during the following discussion, in conjunction with the accompanying drawings, in which:

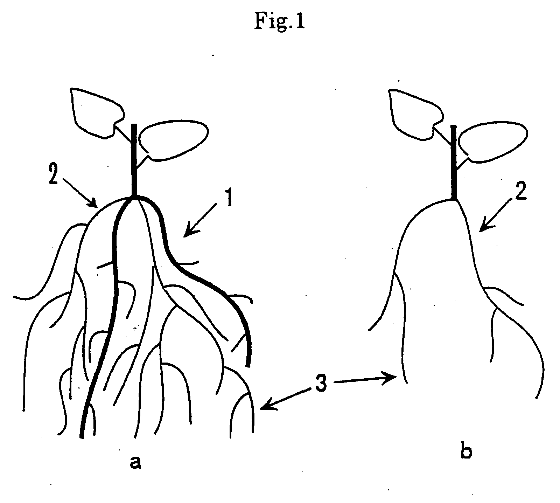

Fig. 1 schematically shows roots of plantlets of sweet potato:
   (a) Plantlets of sweet potato resulting from the production method of the present invention.
   (b) Plantlets of sweet potato obtained in comparative examples.

### DETAILED DESCRIPTION OF THE PREFERED EMBODIMENTS

Embodiments of the present invention will be described in detail hereinafter with reference to the appended drawings.

The method of the present invention is a method for producing plantlets of sweet potato with formed storage roots, characterized by performing tissue culture of explants of sweet potato by using a support material comprising vermiculite and cellulose fibers.

In common methods used for the culture of plantlets of sweet potato, for example, the culture method which utilizes a gelling agent such as agar, the plantlets do not develop storage roots that eventually grow into potatoes, and they develop only main roots and lateral roots (see Fig. 1). Therefore, these plantlets, which have not yet formed storage roots, form new roots that are storage roots, after they are transplanted into nursery soil. As a result, this adds a delay in storage root formation, thus causing the maturation of potatoes to take a greater amount of time.

In contrast, according to the present invention, storage roots, which are to become potatoes, are formed in plantlets. Therefore, when plantlets of sweet potato produced according to the present invention are transplanted to nursery soil, matured sweet potatoes are formed very quickly.

In the present invention, the term" plantlets of sweet potato" refers to cultured seedlings obtained via tissue culture. As well, the term "explants of sweet potato" refers to a part of plant body, which can develop into a plantlet of sweet potato through tissue culture. For example, the explant can consist of a node with leaves, which can be obtained by culturing sweet potato cells or tissue, or a stem piece obtained from a stem having multiple nodes by cutting the stem node by node.

In the present invention, there are no particular restrictions on the specific types of vermiculite, and vermiculite subjected to various types of treatments may be used. Examples of such treatments include chemical and physical treatments of vermiculite such as heating treatment, cooling treatment, purification treatment, swelling treatment, pulverization treatment, granulating treatment, impregnation treatment, coating treatment and so forth.

There are also no particular restrictions on the types of cellulose fibers utilized in the present invention. However, the use of pulp-like fibers is preferable. The cellulose fibers that can be utilized include cotton lint, cotton linter, softwood cellulose, hardwood cellulose, bast cellulose, hemp cellulose, regenerated cellulose, bacterial cellulose and mixtures of these cellulose types.

As well, cellulose fibers that have been subjected to various types of treatments beforehand may be utilized. Examples of such treatments include chemical and physical treatments such as heating treatment, cooling treatment, purification treatment, decrystallization treatment, swelling treatment, treatment for reducing the degree of polymerization, treatment for derivatization, cross-linking treatment, treatment for converting crystal form, regeneration treatment by dissolution, pulverization treatment, granulating treatment, impregnation treatment, coating treatment and so forth.

In the method for producing plantlets of sweet potato according to the present invention, the weight ratio of vermiculite to cellulose fibers used as a support material is preferably 5:95 to 95:5, especially preferably 70:30. When explants are cultured under conditions in which vermiculite content is too high as compared to cellulose content or cellulose content is too high compared to vermiculite content, plantlets may not develop storage roots.

The support material may optionally be added with activated carbon, coconut shell fiber, perlite, smoked charcoal, peat moss and so forth.

The tissue culture of the present invention is preferably performed in a medium (culture broth) that does not contain saccharides. The saccharides include, for example, carbon sources such as sucrose, glucose and fructose.

By culturing plantlets in a medium containing no saccharides, saprophytic contamination can be avoided, which is likely to occur when a saccharide is added to the medium. In addition, explants cultured in a saccharide-free medium can be tissue-cultured consistently under an autotrophic growth condition, instead of under a heterotrophic growth condition.

For the culture of the present invention, there can be used a medium containing inorganic nutrients, for example, macroelements such as nitrogen, phosphorus, potassium, magnesium and calcium, and microelements such as iron, manganese, copper and zinc. Furthermore, the medium can also contain vitamins such as nicotinic acid and thiamin hydrochloride, organic nutrients such as amino acids, growth regulators and so forth, but these are not essential components. As a specific example of the medium, MS medium can be mentioned.

During the tissue culture of the present invention, it is preferable to maintain the concentration of carbon dioxide gas inside the culturing environment to be 400 to 3,000 µmol/mol throughout the duration of the culture period.

The reason why the concentration of carbon dioxide gas is maintained within these specific limits, is that photosynthesis of plantlets may not be sufficient if the carbon dioxide concentration is too low. As well, an overly high concentration of carbon dioxide gas may be harmful to the plantlets.

By using a medium containing no saccharides and maintaining the carbon dioxide gas concentration inside the culturing environment within 400 to 3,000 µmol/mol during the culture of explants of sweet potato as described above, autotrophic growth of the plants can be enhanced. Therefore, even when the plantlets are transferred into an autotrophic growth condition, for example, by transplanting them to the outside of culture vessels or into nursery soil, there is no harsh environmental change. Therefore, there can be produced plantlets that can be acclimatized easily, or may even require no acclimatization, and exhibit good growth.

The concentration of carbon dioxide gas can be controlled by measuring the concentration in the culture vessel or inside the culture room and supplying the gas required for compensating insufficient concentration compared with the defined concentration into the culture vessel or the culture room.

There are no particular restrictions on the vessels used for the culture, but it is preferable to utilize vessels showing gas permeability in order to increase ventilation. For example, a gas-permeable film can be utilized for a lid of the culture vessel to increase aeration. In addition, it is preferable to use a culture vessel at least a part of which is transmissible to light to allow photosynthesis of sweet potato explants. For example, a culture vessel that has a transparent lid can be utilized.

As for other culture conditions such as temperature, type of light, light intensity, direction of light irradiation, light and dark cycle, relative humidity, amount of medium solution, concentration of medium solution and so forth, suitable conditions can be selected by confirming them through experiments.

### Examples

The invention will be described more specifically hereafter with reference to the following examples.

Sweet potato cultured according to a conventional method was cut into nodes each containing one leaf, and four of the single nodes were each cultured inside pot-shaped plastic containers having a volume of 485 cm³. Into each container was placed 45cm³ of MS (Murashige and Skoog, Physiol. Plant., 15, 473-497 (1962)) medium containing no vitamins, no hormones and no saccharides, and a molded support material comprising vermiculite (TG-4, made by Showa Vermiculite Ltd.) and hardwood craft pulp (product name: Pontiac) with various mixing ratios (as shown in Table 1) as a support material.

Then, each of the nodes of sweet potato was placed on the above support material and cultured for 30 days in total under a photoautotrophic culture condition. The conditions of this culture environment consisted of a temperature in the culture containers of 28°C, relative humidity of 80%, light irradiation intensity from a white fluorescent lamp of 150 µmol/m²/s, irradiation period of 16 hours/day, and carbon dioxide gas concentration in incubator of 1,400 µmol/mol.

In Examples 1-6, culture was performed by using support materials comprising vermiculite and the pulp with a ratio of 90:10 to 10:90.

In Comparative Example 1, culture was performed in the same manner as in Examples, except that a support material containing vermiculite alone was used.

In Comparison Example 2, culture was performed in the same manner as in Examples, except that a 0.8 wt% agar medium (Kanto Kagaku) was used as medium.

The measurement results of fresh weights and dry weights of stems, leaves and roots of sweet potato plantlets obtained by culture for 30 days under the conditions described above are shown in Table 1.

**Table 1**

| | V:P ratio in support material | Fresh weight (g) | | | Dry weight (g) | | |
|---|---|---|---|---|---|---|---|
| | | Stem | Leaf | Root | Stem | Leaf | Root |
| Example 1 | V:P/90:10 | 126±24.3 | 983±87.7 | 673±122 | 9.9±2.6 | 82.9±5.8 | 61.4±16.9 |
| Example 2 | V:P/80:20 | 173±46.5 | 1225±178 | 730±78.2 | 12.4±1.4 | 96.1±14.7 | 68.2±13.9 |
| Example 3 | V:P/70:30 | 214±49.8 | 1443±274 | 732±82 | 17.3±2.6 | 111.4±26.5 | 70.1±9.9 |
| Example 4 | V:P/50:50 | 174±45.2 | 1127±147 | 681±92.4 | 13.4±2.1 | 90.5±12.8 | 60.9±9.1 |
| Example 5 | V:P/30:70 | 172±47.1 | 1168±104 | 700±36.1 | 12.6±1.3 | 101±8.3 | 61.6±5.5 |
| Example 6 | V:P/10:90 | 167±15.8 | 1130±155 | 561±93.1 | 12.1±2.4 | 89.7±12.8 | 44.6±6.2 |
| Comparative Example 1 | V:P/100:0 | 90.5±16.7 | 561±97.2 | 387±12.7 | 7.1±1.3 | 47.9±10.6 | 31.1±4.9 |
| Comparative Example 2 | Agar | 106±38 | 623±134 | 307±112* | - | - | - |
| V: Vermiculite P: Pulp | | | | | | | |

| | | | | | | | |
|---|---|---|---|---|---|---|---|
| *: Submerged root | | | | | | | |

As clearly demonstrated by the results shown in Table 1, both of the fresh weights and dry weights were significantly increased in Examples 1-6 compared with Comparative Examples 1 and 2. In particular, the support material of Example 3 having the V:P mixing weight ratio of 70:30 showed marked effect. Thus, it was demonstrated that the plantlets of sweet potato produced by the present invention showed more excellent growth.

Furthermore, among the roots of the plantlets of sweet potato produced in Examples 1-6, there were many purple roots, and these roots showed organic differentiation into storage roots 1 (Fig. 1a). In contrast, the roots of the plantlets of sweet potato produced in Comparative Examples 1 and 2 did not have storage roots, and had only main roots 2 and lateral roots 3, as shown in Fig. 1b.

When the plantlets produced in Examples 1-6 were transplanted directly into nursery soil, the storage roots grew into potatoes.

On the other hand, when the plantlet produced in Comparative Example 1 was similarly transplanted directly into nursery soil, storage roots were formed separately from the main root and lateral roots, and thereafter the storage roots grew into potatoes.

When the plantlet produced in Comparative Example 2 was transplanted directly into nursery soil, it withered and died.

According to the present invention, it is possible to produce plantlets of sweet potato, which show excellent growth and have storage roots formed during their culture.

The plantlets of sweet potato produced by the method of present invention show excellent growth, and are easily acclimatized, or may not even require acclimatization when they are transplanted to nursery soil. Furthermore, because they already possess storage roots, they show much faster mature of potatoes after transplantation into nursery soil compared with conventional plantlets.

Having thus described the present invention, it will be obvious that the same may be varied in various ways. Such variations are not to be regarded as departure from the spirit and scope of the invention, and all such modifications would be obvious for one skilled in the art intended to be included within the scope of the following claims.

## Claims

1. A method for producing plantlets of sweet potato with formed storage roots, wherein tissue culture of explants of sweet potato is performed by using a support material comprising vermiculite and cellulose fibers.

2. The method for producing plantlets of sweet potato according to claim 1, wherein the vermiculite and the cellulose fibers are used in a weight ratio of 5:95 to 95:5.

3. The method for producing plantlets of sweet potato according to claim 1, wherein the culture is performed in a medium not containing a saccharide.

4. The method for producing plantlets of sweet potato according to claim 1, wherein the culture is performed while maintaining carbon dioxide gas concentration in culture environment to be 400 to 3,000 µmol/mol.
